# EUROPEAN PATENT APPLICATION

(11) **EP 2 517 678 A1**
(43) Date of publication of application: **31.10.2012**
(21) Application number: 11188783.2
(22) Date of filing: 11.11.2011
(51) Int. Cl.: A61F 9/00, A61M 11/00

(54) **Ophthalmic ultrasonic nebulizer device**

(30) Priority: 29.04.2011 TW 100115229
(71) Applicant: Middleland Sensing Technology Inc., HsinChu City 30013 (TW)
(72) Inventor: Hsiao, Hsiung, HsinChu City 30013 (TW)
(74) Representative: Reichert, Sabine

(57) **Abstract**

An ophthalmic ultrasonic nebulizer, relying on a built-in ultrasonic nebulizer module to deliver therapeutic pharmaceuticals or preparations or protective formulations through fine mist sprayed directly into the eyes, and fitted with a removable internal chamber for holding pharmaceutical preparations in a conveniently replaceable pre-filled container, so users can directly replace a new container after the liquid has been depleted, thus avoiding risks of contamination during refilling or prolonged use of a container, preventing internal bacterial growth leading to contamination within the container vessel, and ensuring the mist stream generated by the ultrasonic nebulizer device remains pure. The ophthalmic ultrasonic nebulizer module can rely on the most optimal small sized battery to power the device, allowing convenient use and carrying.

## Description

### FIELD OF THE INVENTION

The present invention relates to a liquid nebulizer, and more particularly to an ophthalmic ultrasonic nebulizer.

### BACKGROUND OF THE INVENTION

The prior art discloses an eye-care device, which delivered therapeutic liquids after a steam mist generation process, spraying the therapeutic mist stream directly onto the eyes, and since the eye-care device relied on storage bottles, whenever the time of storage was excessive, this facilitated bacterial growth contamination, so to resolve this problem, the prior art included installation of an antibacterial reaction chamber, with a UV production unit in the chamber, to disinfect the liquid mist in the reaction chamber, thereby ensuring a bactericidal effect. But, consequent the addition of the UV unit to the reactive chamber, the entire eye-care device had to be placed in other spaces, such that given the inherent size constraints of the eye-care device to further minimization, the appearance became quite dull and unappealing, while also resulting in a shortcoming with the device being inconvenient to carry, and a complicated eye-care device design which increased production overhead costs.

Thus, the present invention took into consideration the need for ensuring an efficacious anti-contamination design, while also ensuring convenient carrying for users, and hereby proposes an ophthalmic ultrasonic nebulizer device, which achieves amelioration for the aforementioned deficits in the existing art which failed to adequately account for these considerations.

### SUMMARY OF THE INVENTION

An object of the present invention is to provide an ophthalmic ultrasonic nebulizer device, deploying replaceable containers for the pharmaceutical liquids or eye cleaning solutions, ensuring heightened hygiene when adding more pharmaceutical liquids or eye cleaning solutions, thus avoiding bacterial growth otherwise associated with reuse of a common container which might occur.

Another object of the present invention is to provide an ophthalmic ultrasonic nebulizer device, with a streamlined structural design, thereby reducing production overhead costs while allowing for an aesthetically pleasing sophisticated easy carry design for users.

The aforementioned description provides the present invention's main device functionality, while the present invention can additionally achieve many other effects, which shall not be otherwise explicitly relayed in detail here.

The invention proposes an ophthalmic ultrasonic nebulizer device including a device body structure that houses and holds a power supply unit, an ultrasonic nebulizer module and a replaceable container. The device body structure includes a power on/off switch. The replaceable container contains liquid, the liquid is contact with the ultrasonic nebulizer module, and the power supply unit for the ultrasonic nebulizer module producing vibrations, thereby stimulating and releasing the liquid in fine spray mist.

In an embodiment of the invention, the ultrasonic nebulizer module includes a vibration module, which is contact with the liquid. The vibration module generates vibrations through ultrasonic frequency.

In an embodiment of the invention, the vibration module is a replaceable vibration module.

In an embodiment of the invention, the device body structure includes an opening, from which particles of the fine spray mist may be released.

In an embodiment of the invention, the power supply unit includes a rechargeable battery or a disposable battery.

In an embodiment of the invention, the device body structure includes a power connection unit, which permits electrical connectivity to an external power source, with use of the rechargeable battery thereby permitting recharging.

In an embodiment of the invention, the liquid may be composed of eye drops, eye wash solution, saline solution, or other specialized liquid for the eyes.

In an embodiment of the invention, the device body structure has at least one adjustable button, permitting adjustment to the ultrasonic vibration module's (ultrasonic) frequencies.

In an embodiment of the invention, the replaceable container may be comprised of a disposable material, such as plastic or glass.

In an embodiment of the invention, the replaceable container may include an absorbent element, permitting absorbance of the liquid through to the ultrasonic nebulizer module, facilitating contact with the ultrasonic nebulizer module, ensuring efficacious vibration activity to perform aerosolization, atomization, nebulization, or the like.

In an embodiment of the invention, a size of each of the particles is ranged from 1 µm to 15 µm.

In consequence to the invention's design providing use of the replaceable container, the invention can permit exhausted eye drop liquid to be replaced with a new replaceable container, avoiding the user having to engage in refilling procedures, thereby reducing the concomitant risk of the ophthalmic ultrasonic nebulizer device output containing contaminated mist. Thus, this invention eliminates the need for space for the installation of disinfection devices, and also thus reduces the production overhead costs, and allows a more streamlined and compact design.

### BRIEF DESCRIPTION OF THE DRAWINGS

The present invention will become more readily apparent to those ordinarily skilled in the art after reviewing the following detailed description and accompanying drawings, in which:

FIG 1 is a schematic three-dimensional view of an ophthalmic ultrasonic nebulizer device according to a first embodiment of the present invention.

FIG. 2 is a schematic cross section view of the ophthalmic ultrasonic nebulizer device according to the first embodiment of the present invention.

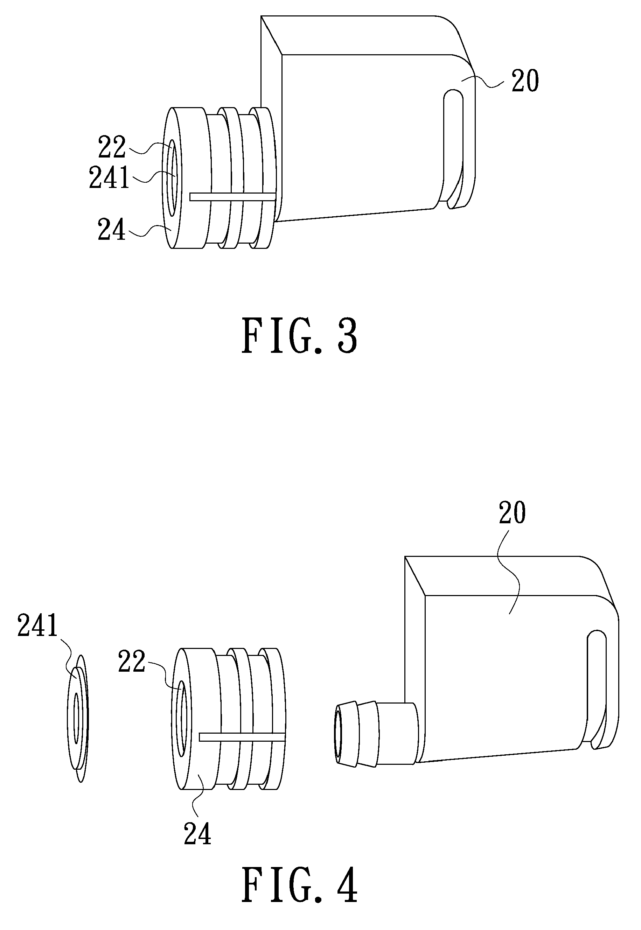

FIG 3 is a schematic view showing relatives of positions of a replaceable container and an ultrasonic nebulizer module according to the first embodiment of the present invention.

FIG. 4 is a schematic exploded view of FIG 3.

FIG 5 a schematic view of using the ophthalmic ultrasonic nebulizer device of the first embodiment of the present invention.

FIG 6 is a schematic three-dimensional view of an ophthalmic ultrasonic nebulizer device according to a second embodiment of the present invention.

FIG 7 shows the internal structural diagrams for the second embodiment of the present invention.

### DETAILED DESCRIPTION OF PREFERRED EMBODIMENTS

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

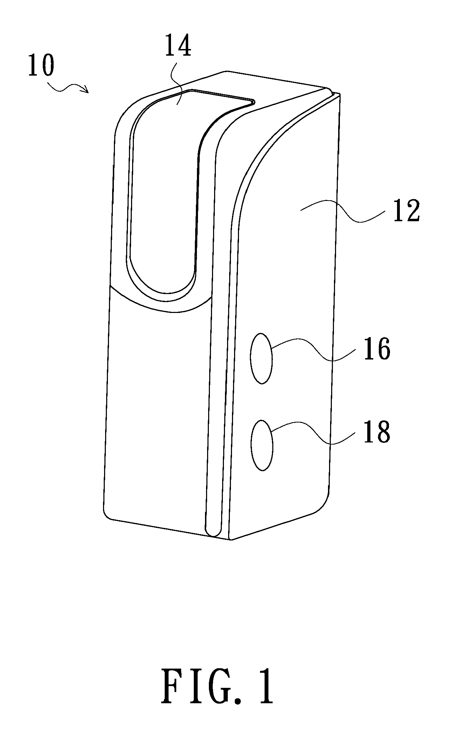

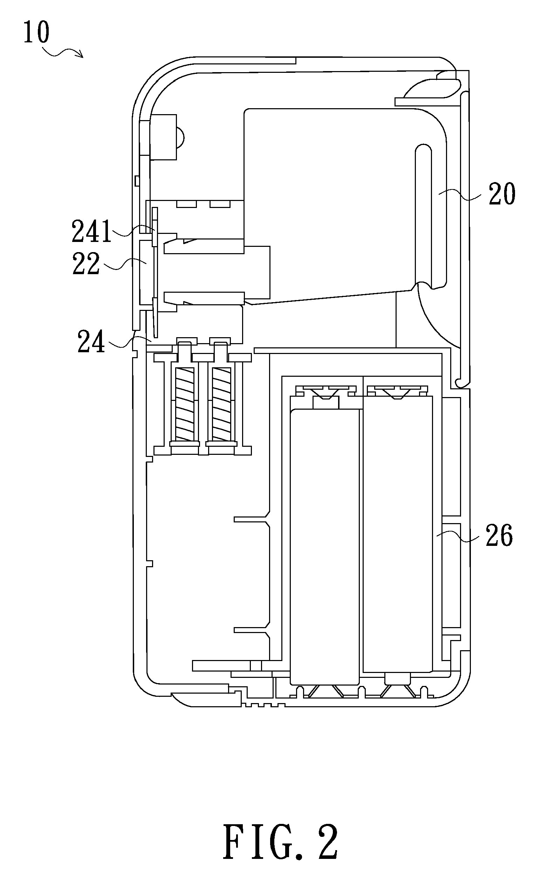

Referring to FIG. 1, the first embodiment of the present invention reveals an ophthalmic ultrasonic nebulizer device 10 with a device body structure. The device body structure includes a shell 12, and the shell 12 may be a square shell. The shell 12 has a cover 14, and the shell 12 has two buttons 16, 18 thereon. The button 16 may be power on/off switch, while the button 18 may be for adjusting mist stream release volume of the ophthalmic ultrasonic nebulizer device 10 or mist stream generation frequency of the ophthalmic ultrasonic nebulizer device 10. The shell 12 has an opening 22 (as shown in FIG 2) covered by the cover 14. The opening 22 is used to release the fine mist stream generated by an ultrasonic nebulizer module 24 of the ophthalmic ultrasonic nebulizer device 10. The present embodiment uses ultrasonic vibrations to generate fine mist streams from liquid such as eye drops, yielding aerosolized particles. A size of each of the particles may be less than 15 µm, or the present embodiment may even control the size of the particle to even less than 3µm, or to even reach 1 µm. Such sized particles permit even distribution across the user's corneal surfaces, and ensure optimally therapeutic efficacy for the eye drops or eye protecting liquids.

Referring to FIG 2, the ophthalmic ultrasonic nebulizer device 10 further includes a container 20 and a power supply unit 26, wherein the container 20, the ultrasonic nebulizer module 24 and the power supply unit 26 are disposed in the shell 12. The container 20 may be a replaceable container, comprised of disposable material, such as plastic or glass, which may be used for the replaceable container, while the overall weight of the ophthalmic ultrasonic nebulizer device 10 is very light, so users are facilitated with a convenient mobile device; while use of replaceable glass container may not only permit users to readily visually determine whether to replace the container, but also permit users to determine when to remove the container for cleaning purposes. The container 20 contains special eye drop liquid for use on the eyes, eye cleaners, saline solution or other specialized liquid for the eyes. Moreover, the ultrasonic nebulizer module 24 contains a vibration module 241, and the vibration module may be a replaceable vibration module. The power supply unit 26 may contain non-rechargeable batteries, or contain at least one rechargeable battery. While with use of the rechargeable battery, the shell 12 of the ophthalmic ultrasonic nebulizer device 10 may be provided with a power connection unit, such as a universal serial bus (USB). The power connected unit is configured to be connected to an external power source, thereby facilitating direct recharging of the rechargeable battery.

Referring to IGS. 3 and 4, the container 22, the ultrasonic nebulizer module 24, and the vibration module 241 are interconnected, with the container 20 which extends forward to within the ultrasonic nebulizer module 24. The vibration module 241 of the ultrasonic nebulizer module 24 may be the replaceable vibration module, and the ophthalmic ultrasonic nebulizer module 10 contains the power supply unit 26 to activate the ultrasonic nebulizer module 24. The vibration module 241 relies on ultrasonic frequency to generate vibration, thereby stimulating the liquid inside the container, among which the vibration module 241 may directly contact with the liquid in the container 20, or an absorbent element may be used to guide the liquid to contact with the vibration module 241. The present embodiment relies on the vibration module 241 conjunctively with ultrasonic frequency generation to stimulate the liquid to nebulized state, among which the absorbent element may be comprised of absorbent cotton or composites.

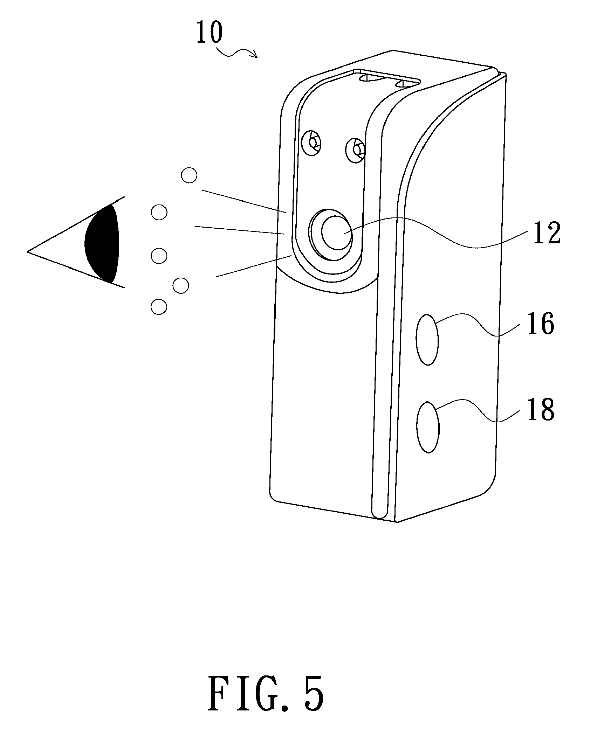

Referring to FIG. 5, when the power on/off switch 16 of the ophthalmic ultrasonic nebulizer device 10 is turned on for permitting generation of fine mist thereafter, users moves the ophthalmic ultrasonic nebulizer device 10 close to their eyes, so that the eye is placed within range of the fine mist stream, the fine mist liquid particles enjoying direct corneal contact, permitting cleaning of the eyes, sterilization or relief to the eyes and concomitant therapeutic effects.

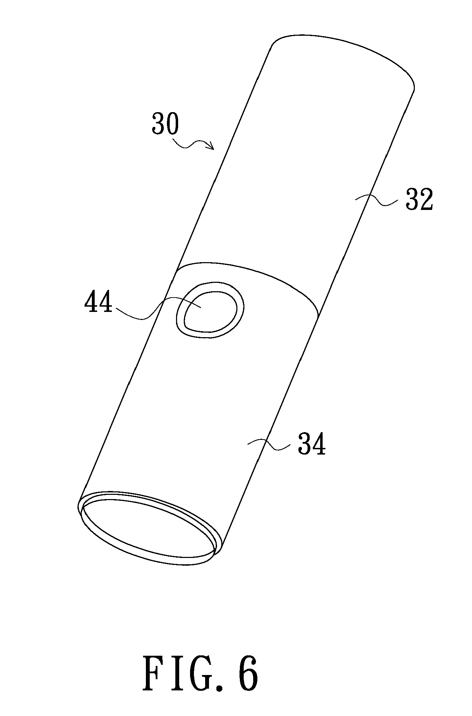

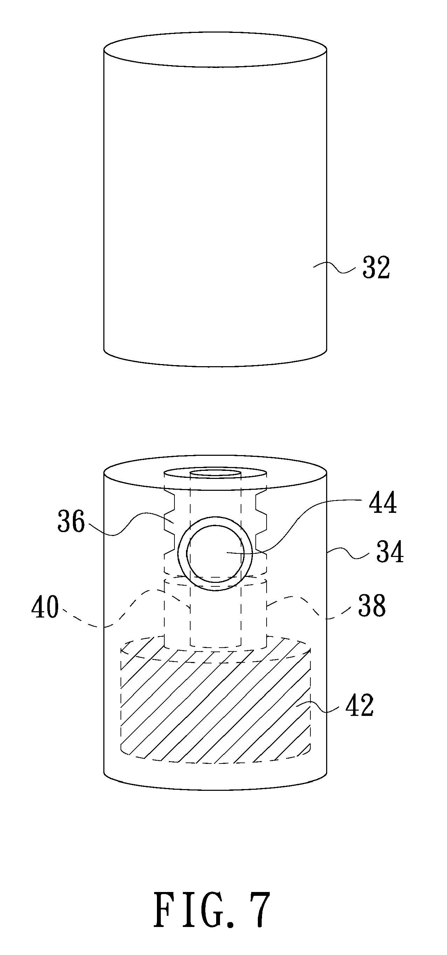

Referring to FIGS. 6 and 7, the ophthalmic ultrasonic nebulizer device 30 of the second embodiment includes a cylindrical device body structure, containing thereon a power on/off switch 44. The device body structure may be composed of the upper shell 32 and primary shell 34,wherein the primary shell 34 is equipped with a ultrasonic nebulizer module 36, a power supply unit 38 and a container 42. The container 42 may be a replaceable container, and may have an absorbent element 40 to guide liquid in the container 42 to the ultrasonic nebulizer module 36. The absorbent element 40 is located betwixt the replaceable container 42 and ultrasonic nebulizer module 36. The liquid is comprised of specialized ophthalmic use eye drop liquid, and the power supply unit 38 provides power to the ultrasonic nebulizer module 36, thereby immediately releasing the nebulized fine mist stream.

In summary of the aforementioned, the present invention's ophthalmic ultrasonic nebulizer device features a replaceable container, hence, users may purchase commercially available replaceable eye drop liquid containers, and after exhaustion of the contained liquid, the user may immediately replace the exhausted container with a replaceable new one, thereby ensuring normal use can maintain the hygienic state of the liquid within the container interior, while also, in conjunction with the present invention's ultrasonic nebulizer module, use with replaceable vibration modules, permits further reductions of the possible risk of contamination of the eye drops, thus strengthening the post-nebulization liquid's hygienic status. As the present invention uses a replaceable container to store the liquid avoiding susceptibility to bacteriological invasion, the invention can reduce the overhead costs and space needs otherwise required for disinfectant equipment, while the ophthalmic ultrasonic nebulizer device can be more compact, allowing users convenience in mobility and use.

While the invention has been described in terms of what is presently considered to be the most practical and preferred embodiments, it is to be understood that the invention needs not be limited to the disclosed embodiment. On the contrary, it is intended to cover various modifications and similar arrangements included within the scope of the appended claims which are to be accorded with the broadest interpretation so as to encompass all such modifications and similar structures.

## Claims

1. An ophthalmic ultrasonic nebulizer device, comprising:
a device body structure including a power on/off switch;
a power supply unit located within the device body structure and electrically connected to the power on/off switch;
at least one ultrasonic nebulizer module located within the device body structure and electrically connected to the power supply unit, the power supply unit being configured to activate the ultrasonic nebulizer module; and
a replaceable container located within or connected with the device body structure, wherein the replaceable container is configured to contain liquid, and the ultrasonic nebulizer module is configured to nebulize the liquid.

2. The ophthalmic ultrasonic nebulizer device according to claim 1, wherein the ultrasonic nebulizer module includes a vibration module, and the ultrasonic nebulizer module relies on ultrasonic frequency to vibrate the vibration module to stimulate release of particles of the liquid.

3. The ophthalmic ultrasonic nebulizer device according to claim 2, wherein the vibration module comprises a replaceable vibration module.

4. The ophthalmic ultrasonic nebulizer device according to claim 1, wherein the device body structure comprises a shell structure, and the shell structure contains an opening used to permit the release of the particles of the liquid.

5. The ophthalmic ultrasonic nebulizer device according to claim 1, wherein the power supply unit comprises at least one rechargeable battery or at least one replaceable battery.

6. The ophthalmic ultrasonic nebulizer device according to claim 1, wherein the power supply unit comprises at least one rechargeable battery, the device body structure comprises a power connection unit configured to be electrically connected to an external power source, so as to recharge the rechargeable battery.

7. The ophthalmic ultrasonic nebulizer device according to claim 1, wherein the liquid comprises ophthalmic use liquid.

8. The ophthalmic ultrasonic nebulizer device according to claim 7, wherein the liquid is selected from one of eye drops, eye wash solution and saline solution.

9. The ophthalmic ultrasonic nebulizer device according to claim 1, wherein the replaceable container is comprised of a disposable material.

10. The ophthalmic ultrasonic nebulizer device according to claim 9, wherein the disposable material is plastic or glass.

11. The ophthalmic ultrasonic nebulizer device according to claim 1, further including an absorbent element contacted with the replaceable container and the ultrasonic nebulizer module, wherein the absorbent element is configured to guide the liquid to contact with the ultrasonic nebulizer module, and the liquid vibrated by the ultrasonic nebulizer module is nebulized to generate the particles.

12. The ophthalmic ultrasonic nebulizer device according to claim 1, wherein a size of each of the particles is ranged from 1 µm to 15 µm.
